Europäisches Patentamt

European Patent Office

Office européen des brevets

(11) Publication number: **0 135 955 B1**

# EUROPEAN PATENT SPECIFICATION

(45) Date of publication of patent specification: **21.11.91**

(51) Int. Cl.⁵: **A61K  31/70**, //(A61K31/70, 31:675)

(21) Application number: **84201131.4**

(22) Date of filing: **02.08.84**

(54) Composition for the treatment of malignant tumours, particularly in animals.

(30) Priority: **26.08.83 IT 6789983**

(43) Date of publication of application:
**03.04.85 Bulletin  85/14**

(45) Publication of the grant of the patent:
**21.11.91 Bulletin  91/47**

(84) Designated Contracting States:
**AT BE CH DE FR GB LI LU NL SE**

(56) References cited:
**EP-A- 0 100 022**

(73) Proprietor: **Spisni, Dino**
**Via Garibaldi, 29**
**I-56017 S. Giuliano Terme (Pisa)(IT)**

Proprietor: **Sasso, Mirella**
**Via Magenta, 3**
**I-54100 Massa(IT)**

Proprietor: **Pellegrini, Giuliano**
**Via Masaggio 25-27**
**I-Firence(IT)**

Proprietor: **Pellegrini, Paola**
**Via Masaggio 25-27**
**I-Firence(IT)**

(72) Inventor: **Spisni, Dino**
**Via Garibaldi, 29**
**I-56017 - S. Giuliano Terme (PISA)(IT)**

(74) Representative: **Lotti, Giorgio**
**c/o Ing. Barzanò & Zanardo Milano S.p.A. Via**
**Cernaia 20**
**I-10122 Torino(IT)**

## Description

Reduced beta-nicotinamide adenine dinucleotide(NADH$_2$) and pyridoxal-5-phosphate are coenzymes respectively of cystine reductase, and cysteine desulphydrase.

It is also well known that cystine and cysteine are involved in tumours and can be found in cancerous animals and that reduced quantities of NADH$_2$ and pyridoxal -5 phosphate are contained in cancerous tissues; the quantity of NADH$_2$ is even more negligeable than that of NAD.

Moreover tests have proven that reduced levels of NAD and NADH$_2$ are to be found in the liver of BR6 rats suffering from mammary tumours depending on or independent of pregnancy, and the content of pyridoxal-5-phosphate in the liver of cancerous rats decreased after intraperitoneal injection of cells of the ascitic hepatoma AH 130.

The decrease in pyridoxal-kinase activity during the first stage of the tumoural formation caused the depletion of pyridoxal-5-phosphate from the liver of cancerous rats and the reaction generating pyridoxal-5-phosphate catalyzed by pyridoxal-kinase was also considerably inhibited by isoniazid (antivitamin B$_6$, carcinogenic factor) . Therefore the content of these two coenzymes proved to be limited in cancerous tissues as well as in the liver of cancerous animals and the administration of both coenzymes could be a benefit for animals.

To verify this assumption studies have been carried out on animals.

A solution of pyridoxal-5-phosphate with pH around 7 was obtained by means of NaOH. The products were lyophilized and sterile solutions were used.

Ten animals underwent this study, 3 to 30 kg heavy, afflicted with various spontaneous malignant tumours. 20 mg beta-NADH$_2$, disodium salt and 8 mg of pyridoxal-5-phosphate (10 and 4 mg resp. in a cat) were administered separately to each animal once a day by intramuscular injection, for a period of time ranging from 20 days to 6 months. Only when the results appeared to have stabilized , were the two coenzymes administered once every two days.

## THERAPY 1

Ten-year-old Irish setter dog, without appetite for many days was found to be very seriously prostrated; aching ,hypertensive abdomen , ascites, dyspnea, impressive signs of Lesoubryriès , general atonicity , emaciated , dull eyes and tremors. The owner asked for euthanasia. X-rays showed an extended hepatic shadow.

A hypodermoclysis of glucosated solutions was administered with no results. Small doses of diuretics let the ascites regress, thus allowing for palpation of a hard, nodous formation in the hepatic area as big as the head of a boy.

All other treatment was suspended and the two coenzymes were administered. For four days the dog , which had not eaten for over ten days vomiting gastric acids from time to time , had been struggling against death. Then it gradually improved, regained its strength , motility, appetite, tonicity and normal vivacity. The bulge volume decreased of over a half and appreciable regressions of the nodular buboes could be ascertained. The tumour turned out to be a lymphosarcoma.

## Therapy 2

Mongrel suffering from a fibrosarcoma sub cutis died after 20 days' treatment with the two coenzymes when the tumour had regressed almost completely . Its death was probably due to an internal hemorrhage.

## Therapy 3

Cat suffering from fibrosarcoma sub cutis , which had formed again after removal.It stood the first two injections of coenzymes well, but showed a malaise on the third day, which grew more serious on the fourth. However, treatment continued and the cat fully recovered with no collateral intervention whatsoever. The first signs of an improvement were noticed on the sixth day: sharp eutrophic improvement, it was hungry and very lively again. The tumour decreased in volume gradually at its fulcrum , until the 23rd day, while nodular metastases regressed and disappeared almost entirely. Then, as from the 24th day of treatment, the neoformation in the treatment fulcrum started growing again back to the conditions, which had been ascertained when treatment had begun, while the 30 nodular metastases continued to reduce until they disappeared. Then the cat's owners asked that the tumour be removed , which was done .

The tumour was submitted to a histological examination. No more was heard about the cat.

## Therapy 4

12-year-old dog, suffering from stomach cancer (epithelioma) It was submitted to a 25 days' treatment with the coenzymes and had remarkably improved. Then it was submitted to surgery as requested by its owners.

## Therapy 5

An eight-year-old setter Gordon , with a widespread paraocular neoformation up to the upper mandibula (neurinoma, schwannoma) . It was treated for several months. The dog's pain was relieved after the first few injections of coenzymes; had the animal neoformation not been seen, its behaviour would have been reputed normal throughout the treatment. One could have thought of an animal with a benign neoformation. The dog was well for several months, precisely until treatment was suspended for a fortnight. Upon this interruption the conditions of the dog grew worse and it was submitted to euthanasia according to its owners' will.

Therapy 6

A ten-year-old boxer with an enormous hepatic cancer ; rather serious general conditions. It was not very easy to appreciate the reduction of the neoplasm upon palpation, as it was a very fat dog and its abdomen was hardly palpable. X-rays would show that the hepatic shadow had decreased. Treatment lasted 29 days and made its general conditions improve sharply and probably reduced the neoplasm. Unfortunately its vet died and no more was heard about this dog.

Therapy 7

Medium-size, male, grizzled Schnauzer, eleven years old.Its conditions were rather precarious, cardiopathy, sharp abdominal proliferation , the nature of which was quite obvious, hard and bulky in the pancreatic area. The radiological diagnosis suggested an almost certain pancreatic neoplasm. The conditions of the dog were really precarious when the therapeutic treatment started with the administration of the two coenzymes after suspending any other treatment. The general conditions of the dog worsened during the first six days of treatment; only on the seventh day did the dog get better, still without administering any other medicine. He began to improve considerably: his appetite was good, he regained his joy of life and even put on a few hectograms. Most important was a gradual reduction in the abdominal neoformation, easily followed by palpation. But his heart was not strong enough any more and the dog died after 24 days' treatment with coenzymes because of a heart failure when the tumour had actually regressed. His owner did not give his permission for autopsy.

Another two dogs afflicted with solid tumours (an osteo sarcoma) were treated with the two coenzymes, and with thymic extract. Anyway it was possible to remark the benefit obtained by the coenzymes.

Material for histologic research has been taken from five out of nine subjects afflicted with different neoplasms either by surgical removal of the cancerours bulk or after their death during autopsy.

The parts to be submitted to histological investigation were taken out and immediately fixed in formalin of Policard, Bouin and Susa. After being left in formalin for a while, they have been included in paraffin and dissected according to normal routine practise. The dissected parts have been coloured for general and particular histological investigation according to the diagnostic goals set for that particular type of neoplasm.

Histologically different neoplasms have been found in the material examined, that is lymphosarcoma in a dog, fibrosarcoma in a dog and a cat, epithelioma in a dog and neurinoma in a dog.

The above mentioned tumour types were easily detected also by studying their general histologic picture , but they had a cytological component, which , even if it was characterized by a remarkable polymorphism and polychromatism with significant nuclear volume, clearly denounced a gradually more remarkable involution in those subjects, which had been submitted to a constant, long-term treatment.

Therefore it has been proven that the various treatments had actually produced some positive effects, with sometimes astonishing results in all animals being treated. The positive results could already be seen 7 to 10 days after beginning treatment and included well-being, regression of metastases , decrease in tumour volume, together with an involution of tumoral cells (see pictures). There was no toxicity; only two animals had trouble perhaps in relation to a metabolism closer to a normal one. When treatment was interrupted , the positive effects decreased, thus proving that treatment should have been continued.

$NADH_2$ has been thought to have the same effect on cystine ionizing radiations (breaking the disulfide bonds, making them react) by acting on cystine reductase, and pyridoxal-5-phosphate was thought to act on cysteine, which formed by acting on cysteine desulfhydrase.

It is well known that if there is a period of high SH-groups in animal tissues during carcinogenesis, subnormal levels of SH groups are found in the grown tumour.

The cystine : cysteine ratio in tumours was increased .

Furthermore pyridoxal-5-phosphate is also a racemase coenzyme , involved in interconversion of d. and l. amino acids and the quantity of d.amino acids is particularly high in tumours. They are also immunosuppressive.

DNA is known to play a role in malignant tumours. On the other hand, a relation between -SH bound to protein and the DNA contained in a few tumoral cells was found , thus leading to the hypothesis that amino acids might play a role in DNA. There are neither macroscopic nor microscopic results as to tumours, one could think that the positive effects should be attributed to the coenzymes administered, lacking in the live cancerous subjects, because NADH$_2$ (and NAD) are coenzymes of about 38 enzymes, and pyridoxal-5-phosphate is an important coenzyme (no less than 22 amino acids are involved in transamination reactions and decarboxylase , phosphorylase and many dehydrase are enzymes depending on pyridoxal-5-phosphate).

It is hard to say whether there are two effects, a general and a specific one, or not. As any specific effect is missing, one could conclude that the general effect could be responsible for the apparent specific effect, that is to say that the malfunction of the liver is primitive and the malignant disease is only a side-effect. This should be a new consciousness , also important for tumour prevention.

Coenzyme deficiency could be responsible for the onset of the malignant disease.

Such a therapy can be explained by the fact that both an excessive quantity of reducing substances and an excess of oxidated substances can provoke the onset of the malignant neoplasm , and the administration of NADH$_2$ , stimulating the production of cysteine from cystine, requires the administration of pyridoxal-5-phosphate too, stimulating the desulfuration of cysteine which would be as harmful as an overproduction of cystine.

On the other hand the administration of pyridoxal-5-phosphate , stimulating the desulfuration of cysteine, requires the administration of NADH$_2$ too, stimulating the production of cysteine, to avoid a harmful overproduction of cystine which would be as harmful as an overproduction of cysteine.

As prothrombin is a protein containing disulfur and being thromboembolic and hemorrhagic disorders one of the major causes of diseases and mortality in patients afflicted with malignant tumours , the proposed therapy required the control of prothrombin , even if NADH$_2$ and pyridoxal-5-phosphate are balancing factors.

Having studied other coenzymes, we have concluded that coenzymes such as thiamine diphosphate, flavin-adenine dinucleotide (FAD), reduced nicotinamide adenine dinucleotide phosphate (NADPH$_2$) , coenzyme A (CoA) , and folinic acid , essential for cell differentiation and co-factors for several enzymes , are deficient not only in tumours, but also in the tissues of tumourous animals. In malignant tumours there is certainly a combined deficiency of antagonist coenzymes, needed for cell differentiation.

The inapparent specific physical reactions to a deficiency in essential co-factors can be explained by a contemporary deficiency in antagonist cofactors.

The coenzymes we have taken into consideration , involved in malignant tumours originate from vitamins necessary for the immunologic response, together with vitamin A.

As malignant tumours cause immunosuppression and immunosuppression causes malignant tumours, the significance of the results is evident. The coenzymes missing in the malignant disease can be responsible not only for immunosuppression in malignant tumours, but even of the onset of the malignant tumour itself, in that the differentiation is suppressed and the administration of these coenzymes of that of substances promoting them, can at least contribute to prevent immunosuppression and find a remedy for malignant tumours.

## Claims

1. Pharmaceutical product for the treatment of malignant tumours, characterized by the fact that they include the following two compounds: reduced beta-nicotinamide adenine dinucleotide (NADH$_2$) disodium salt and pyridoxal-5-phoshate, respectively coenzymes of cystine reductase and of cysteine desulfhydrase.

2. Pharmaceutical product as per claim 1, characterized by the fact that the above mentioned compounds are presented for administration simultaneously or separately.

3. Pharmaceutical product as per claim 1, characterized by the fact that each administration includes 5 to 40 mg beta NADH$_2$ disodium salt and 2 to 20 mg pyridoxal-5-phosphate.

## Revendications

1. Produit pharmaceutique pour le traitement de tumeurs malignes, caractérisé en ce qu'il comprend les deux composés suivants: beta-nicotinamide adenine dinucleotide (NADH$_2$) réduite, sel disodique et pyridoxal-5-phosphate, respectivement coenzymes de cystine réductase et de cysteine désulphydrase.

2. Produit pharmaceutique selon la revendication 1 caractérisé en ce que les composés susdits sont présentés pour administration simultanée ou séparée.

3. Produit pharmaceutique selon la revendication 1 caractérisé en ce que chaque administration comprend 5 à 40 mg de beta NADH$_2$, sel disodique et 2 à 20 mg de pyridoxal-5-phosphate.

## Patentansprüche

1. Arzneimittel zur Behandlung von bösartigen Geschwülsten, dadurch gekennzeichnet, daß es folgende zwei Komponenten umfaßt: reduziertes Beta-Nikotinamid Adenin Dinucleotid ($NADH_2$), Dinatriumsalz und Pyridoxal-5-Phosphat bzw. Koenzyme von Zystin-Reduktase und Zystein-Desulfhydrase.

2. Arzneimittel nach Anspruch 1, dadurch gekennzeichnet, daß besagte Komponenten zur gleichzeitigen oder getrennten Verabreichung angeboten werden.

3. Arzneimittel nach Anspruch 1, dadurch gekennzeichnet, daß jede Verabreichung 5 bis 40 mg von Beta $NADH_2$, Dinatriumsalz und 2 bis 20 mg von Pyridoxal-5-Phosphat umfaßt.